# FASCICULE DE BREVET EUROPEEN

(11) **EP 0 703 205 B1**
(45) Date de publication et mention de la délivrance du brevet: **28.01.1998**
(21) Numéro de dépôt: 95401917.0
(22) Date de dépôt: 21.08.1995
(51) Int. Cl.: C07C 17/20, C07C 19/08

(54) **Synthèse du 1,1,1,3,3-pentafluoropropane**
Synthese von 1,1,1,3,3-Pentafluorpropan
Synthesis of 1,1,1,3,3-pentafluoropropane

(30) Priorité: 26.09.1994 FR 9411451
(43) Date de publication de la demande: 27.03.1996
(73) Titulaire: ELF ATOCHEM S.A., 92800 Puteaux (FR)
(72) Inventeur: Wendlinger, Laurent, F-69230 Saint Genis Laval (FR); Lantz, André, F-69390 Vernaison (FR)
(74) Mandataire: Leboulenger, Jean

(56) Documents cités:
- EP-A- 0 522 639

## Description

La présente invention concerne la préparation du 1,1,1,3,3-pentafluoropropane (connu dans le métier sous la désignation HFA 245fa) et a plus particulièrement pour objet un procédé de préparation du 1,1,1,3,3-pentafluoropropane, en une seule étape, à partir de 1,1,1,3,3-pentachloropropane.

En raison de l'appauvrissement de la couche d'ozone, une limitation de la production des chlorofluorocarbones entièrement halogénés (CFC) a été programmée dès 1986 (protocole de Montréal). Une dernière réactualisation, en 1992 (convention de Copenhague), a même arrêté le principe de l'abandon total de ces produits à la fin de l'année 1995. La commission européenne a fixé la barre encore plus haut en imposant un arrêt complet de la production dés la fin de l'année 1994.

Les recherches pour trouver des remplaçants à ces composés se sont focalisées, dans un premier temps, sur des produits contenant des atomes d'hydrogène (HCFC), puis sur des produits ne contenant plus de chlore : les hydrofluorocarbones (HFC).

Parmi ceux-ci, un intérêt croissant pour les composés en C₃ (dont le HFA 245fa) semble se manifester. Par exemple, l'utilisation de 1,1,1,3,3-pentafluoropropane est évoquée dans différentes demandes de brevet, notamment comme agent d'expansion pour mousse (JP 5239251), comme agent d'expansion, gaz propulseur et solvant de nettoyage pour l'industrie électronique (DD 298419) et enfin comme fluide caloporteur (JP 2272086).

Knunyants *et al. (Izvest. Akad. Nauk. S.S.S.R., Otdel. Khim. Nauk*. 1960, 1412-1418 ; C.A. 55 : 349c et *Kinet. Katal.* 1967, *8(6)*, 1290-1299 ; C.A. 69 : 3510n) décrivent la synthèse du 1,1,1,3,3-pentafluoropropane par hydrogénation catalytique du 1,1,3,3,3-pentafluoro-1-propène (CF₃-CH=CF₂) sur des catalyseurs à base de palladium déposé sur alumine. Cependant, l'obtention du produit de départ n'est pas aisée, et les auteurs précisent bien qu'ils ont été confrontés à des problèmes de sélectivité dus à des réactions de "substitution" des atomes de fluor par des atomes d'hydrogène.

Le brevet US 2942036 revendique un procédé d'hydrogénolyse du 1,2,2-trichloropentafluoropropane (CF₃-CCl₂-CClF₂) permettant d'obtenir le 1,1,1,3,3-pentafluoropropane. L'inconvénient de cette synthèse réside dans la préparation du produit halogéné de départ, qui nécessite plusieurs étapes mettant en oeuvre des réactions de chloration et de fluoration.

Burdon *et al*. (*J. Chem. Soc. C*, 1969, *13*, 1739-1746) mentionnent également l'obtention de 1,1,1,3,3-pentafluoropropane (parmi 30 autres composés) lors de la réaction du tétrahydrofuranne avec le trifluorure de cobalt. Cette réaction de laboratoire est peu sélective; elle paraît également difficilement extrapolable industriellement.

La présente invention a donc pour but de fournir un procédé permettant la synthèse de 1,1,1,3,3-pentafluoropropane avec une bonne sélectivité, à partir de 1,1,1,3,3-pentachloropropane, en une seule étape.

Le procédé selon l'invention consiste en une réaction de fluoration du 1,1,1,3,3-pentachloropropane par l'acide fluorhydrique anhydre, en phase liquide et en présence d'un catalyseur.

Le 1,1,1,3,3-pentachloropropane, utilisé comme produit de départ, peut être facilement préparé en une seule étape par réaction du tétrachlorométhane (CCl₄) avec le chlorure de vinyle (CH₂=CHCl), deux produits industriels largement disponibles.

Le catalyseur de la réaction de fluoration du 1,1,1,3,3-pentachloropropane par l'acide fluorhydrique anhydre, en phase liquide, est choisi parmi les dérivés des métaux appartenant aux groupes principaux IIIa, IVa et Va et aux sous-groupes IVb, Vb et VIb. Ces composés métalliques peuvent être utilisés seuls ou en mélange. Parmi les éléments sélectionnés dans les colonnes de la classification périodique, on peut retenir plus spécialement le titane, le niobium, le tantale, le molybdène, le bore, l'étain et l'antimoine. Les espèces contenant de l'antimoine conviennent particulièrement bien. Comme dérivé métallique, on peut utiliser les oxydes, les oxyhalogénures et les halogénures. On choisit plus particulièrement les halogénures, avec une préférence pour les chlorures, les fluorures et les chlorofluorures. Le pentachlorure d'antimoine (SbCl₅) convient spécialement bien; son utilisation conduit à une transformation importante du 1,1,1,3,3-pentachloropropane et une sélectivité élevée en 1,1,1,3,3-pentafluoropropane.

La quantité de catalyseur mise en oeuvre dans le procédé peut varier dans de larges limites. Elle est d'au moins 0,005 mole de catalyseur par mole de 1,1,1,3,3-pentachloropropane et n'excède pas 0,5 mole de catalyseur par mole de 1,1,1,3,3-pentachloropropane. De préférence, la quantité de catalyseur utilisée est comprise entre 0,02 mole et 0,25 mole de catalyseur par mole de 1,1,1,3,3-pentachloropropane.

Le procédé selon l'invention est mis en oeuvre par chauffage des réactifs. La température de réaction est généralement d'au moins 50 °C et, le plus souvent, elle n'excède pas 150 °C. De préférence, la réaction est effectuée à une température située entre 80 °C et 120 °C.

Selon l'invention, la réaction de fluoration du 1,1,1,3,3-pentachloropropane peut être conduite de manière discontinue ou continue.

En discontinu, on opère dans un réacteur fermé dans lequel tous les réactifs sont introduits au début de l'opération. La pression autogène augmente avec l'avancement de la réaction, pour atteindre une valeur maximale. Selon ce procédé, le rapport molaire entre l'acide fluorhydrique anhydre et le 1,1,1,3,3-pentachloropropane est au minimum de 5 et ne dépasse généralement pas 100. De préférence, la réaction s'effectue avec un rapport molaire entre l'acide fluorhydrique anhydre et le 1,1,1,3,3-pentachloropropane compris entre 10 et 50 et, plus particulièrement, avec un rapport molaire de 20 environ.

En continu, les réactifs sont alimentés régulièrement dans le milieu réactionnel contenant le catalyseur. De manière avantageuse, le réacteur est surmonté d'une colonne permettant d'éliminer constamment une partie ou la totalité des composés légers générés au cours de la réaction (acide chlorhydrique, 1,1,1,3,3-pentafluoropropane) et de condenser les composés plus lourds (catalyseur, acide fluorhydrique, produits chloro-fluorés intermédiaires). La pression de réaction est maintenue à une valeur déterminée, au moyen d'un dispositif de régulation approprié. Cette valeur est définie, d'une part, pour permettre la séparation de l'acide chlorhydrique par distillation et, d'autre part, pour maintenir le milieu réactionnel à l'état liquide; elle varie donc en fonction de la température du milieu réactionnel. La pression de réaction est d'au moins 5 bars et ne dépasse pas 60 bars. De préférence, la pression de réaction se situe dans l'intervalle de pression compris entre 10 et 40 bars. Des pressions comprises entre 12 et 30 bars se sont révélées particulièrement avantageuses.

Les exemples qui suivent illustrent l'invention sans toutefois en limiter la portée. Dans ces exemples, les indications de pourcentages s'entendent en moles, sauf mention contraire.

### EXEMPLE 1

Dans un autoclave en INOX 316L de 800 ml, équipé d'un indicateur de pression, d'une sonde thermométrique, d'un disque d'éclatement et d'un système d'agitation par barreau magnétique, on a introduit successivement 9,5 g de pentachlorure d'antimoine (0,032 mole), 35,5 g de 1,1,1,3,3-pentachloropropane (0,164 mole) et 62,3 g d'acide fluorhydrique anhydre (3,113 moles).

Le réacteur a été chauffé à 120 °C et la pression a augmenté graduellement pour atteindre 42,5 bars. Après 5 heures et demie, le système réactionnel a été ramené à température ambiante et une pression résiduelle de 14 bars a été observée.

Les analyses des différentes phases recueillies ont donné les résultats suivants:
- taux de transformation du 1,1,1,3,3-pentachloropropane: ≥ 99,5 %
- sélectivité en 1,1,1,3,3-pentafluoropropane : 69,0 %

Les autres produits de réaction sont principalement des composés sous-fluorés: C₃H₃Cl₂F₃ (sélectivité: 22,6 %) et C₃H₃ClF₄ (sélectivité: 5,5 %). Ces composés peuvent être avantageusement recyclés au réacteur, pour être transformés en 1,1,1,3,3-pentafluoropropane.

### EXEMPLE 2(COMPARATIF)

Dans le même réacteur qu' à l'exemple 1, on a introduit successivement 33 g de 1,1,1,3,3-pentachloropropane (0,153 mole) et 61,7 g d'acide fluorhydrique anhydre (3,083 moles).

Le réacteur a été chauffé à 120 °C et la pression a augmenté graduellement pour atteindre 27,5 bars. Après 5 heures et demie, le système réactionnel a été ramené à température ambiante et une pression résiduelle de 7 bars a été observée.

Les analyses des différentes phases recueillies ont fourni les résultats suivants :
- taux de transformation du 1,1,1,3,3-pentachloropropane : 99,1 %
- sélectivité en 1,1,1,3,3-pentafluoropropane : 0,05 %

Au cours de cette réaction, deux produits ont été obtenus majoritairement et dans des proportions voisines:
- le 1,1,3,3-tétrachloro-1-fluoropropane (CCl₂F-CH₂-CHCl₂) : 50,7 %
- le 1-chloro-3,3,3-trifluoropropène (CF₃-CH=CHCl) : 45,0 %.

### EXEMPLE 3

Dans un autoclave en INOX 316L de 1000 ml, équipé d'un condenseur (relié à un système indicateur-régulateur de pression), d'un indicateur de pression, d'une sonde thermométrique, d'un disque d'éclatement et d'un système d'agitation par barreau magnétique, on a introduit successivement 9,2 g de pentachlorure d'antimoine (0,031 mole), 66,5 g de 1,1,1,3,3-pentachloropropane (0,307 mole) et 123,0 g d'acide fluorhydrique anhydre (6,147 moles).

Une circulation d'eau (T=20 °C) a été établie dans le condenseur; le réacteur a été chauffé à 120 °C et la pression a été régulée à 20 bars par prélèvement de composés légers. Après 5 heures et demie, le système réactionnel a été ramené à température ambiante; une pression résiduelle de 1,6 bars a été observée.

Les analyses des différentes phases recueillies ont donné les résultats suivants :
- taux de transformation du 1,1,1,3,3-pentachloropropane: ≥ 99,9 %
- sélectivité en 1,1,1,3,3-pentafluoropropane : 91,8 %

Les autres produits de réaction sont principalement des composés sous-fluorés: C₃H₂ClF₃ (sélectivité: 1,4 %), C₃H₃ClF₄ (sélectivité: 0,9 %) et C₃H₃Cl₂F₃ (sélectivité: 0,6 %).

## Revendications

1. Procédé de fabrication du 1,1,1,3,3-pentafluoropropane caractérisé en ce que l'on fait réagir, en phase liquide, du 1,1,1,3,3-pentachloropropane avec de l'acide fluorhydrique anhydre, en présence d'un catalyseur.

2. Procédé selon la revendication 1, dans lequel le catalyseur est choisi parmi les dérivés des métaux des groupes principaux IIIa, IVa et Va et des sous-groupes IVb, Vb et VIb.

3. Procédé selon la revendication 2, dans lequel le catalyseur est un mélange de dérivés métalliques.

4. Procédé selon la revendication 2 ou 3, dans lequel les dérivés métalliques sont choisis parmi les oxydes, les oxyhalogénures et les halogénures.

5. Procédé selon la revendication 4, dans lequel les halogénures sont choisis parmi les chlorures, les fluorures et les chlorofluorures.

6. Procédé selon l'une des revendications 1 à 3, dans lequel le catalyseur est choisi parmi les chlorures, les fluorures et les chlorofluorures d'antimoine et leurs mélanges.

7. Procédé selon la revendication 1, dans lequel le catalyseur est le pentachlorure d'antimoine.

8. Procédé selon l'une des revendications 1 à 7, dans lequel on utilise de 0,005 à 0,5 mole de catalyseur par mole de 1,1,1,3,3-pentachloropropane, de préférence de 0,02 à 0,25 mole de catalyseur par mole de 1,1,1,3,3-pentachloropropane.

9. Procédé selon l'une des revendications 1 à 8, dans lequel la réaction est effectuée à une température allant de 50 °C à 150 °C, de préférence entre 80 °C et 120 °C.

10. Procédé selon l'une des revendications 1 à 9, dans lequel on opère de manière discontinue, sous la pression autogène du mélange réactionnel.

11. Procédé selon la revendication 10, dans lequel le rapport molaire entre l'acide fluorhydrique anhydre et le 1,1,1,3,3-pentachloropropane est compris entre 5 et 100, de préférence entre 10 et 50.

12. Procédé selon l'une des revendications 1 à 9, dans lequel on opère de manière continue sous une pression comprise entre 5 et 60 bars, de préférence entre 10 et 40 bars.

## Claims

1. Process for the manufacture of 1,1,1,3,3-pentafluoropropane, characterized in that 1,1,1,3,3-pentachloropropane is reacted, in liquid phase, with anhydrous hydrofluoric acid in the presence of a catalyst.

2. Process according to Claim 1, in which the catalyst is chosen from the derivatives of metals of main groups IIIa, IVa and Va and of subgroups IVb, Vb and VIb.

3. Process according to Claim 2, in which the catalyst is a mixture of metal derivatives.

4. Process according to Claim 2 or 3, in which the metal derivatives are chosen from oxides, oxyhalides and halides.

5. Process according to Claim 4, in which the halides are chosen from chlorides, fluorides and chlorofluorides.

6. Process according to one of Claims 1 to 3, in which the catalyst is chosen from antimony chlorides, fluorides and chlorofluorides and mixtures thereof.

7. Process according to Claim 1, in which the catalyst is antimony pentachloride.

8. Process according to one of Claims 1 to 7, in which 0.005 to 0.5 mol of catalyst is employed per mole of 1,1,1,3,3-pentachloropropane, preferably from 0.02 to 0.25 mol of catalyst per mole of 1,1,1,3,3-pentachloropropane.

9. Process according to one of Claims 1 to 8, in which the reaction is performed at a temperature ranging from 50°C to 150°C, preferably between 80°C and 120°C.

10. Process according to one of Claims 1 to 9, in which the operation is carried out noncontinuously, at the autogenous pressure of the reaction mixture.

11. Process according to Claim 10, in which the molar ratio of anhydrous hydrofluoric acid to 1,1,1,3,3-pentachloropropane is between 5 and 100, preferably between 10 and 50.

12. Process according to one of Claims 1 to 9, in which the operation is carried out continuously at a pressure of between 5 and 60 bars, preferably between 10 and 40 bars.

## Patentansprüche

1. Verfahren zur Herstellung von 1,1,1,3,3-Pentafluorpropan, dadurch gekennzeichnet, daß man in flüssiger Phase 1,1,1,3,3-Pentachlorpropan in Gegenwart eines Katalysators mit wasserfreier Fluorwasserstoffsäure reagieren läßt.

2. Verfahren nach Anspruch 1, bei dem der Katalysator ausgewählt ist aus Derivaten der Metalle der Hauptgruppen IIIa, IVa und Va und der Nebengruppen IVb, Vb und VIb.

3. Verfahren nach Anspruch 2, bei dem der Katalysator eine Mischung von Metallderivaten ist.

4. Verfahren nach Anspruch 2 oder 3, bei dem die Metallderivate ausgewählt sind aus Oxiden, Oxyhalogeniden und Halogeniden.

5. Verfahren nach Anspruch 4, bei dem die Halogenide ausgewählt sind aus Chloriden, Fluoriden und Chlorfluoriden.

6. Verfahren nach einem der Ansprüche 1 bis 3, bei dem der Katalysator ausgewählt ist aus Antimonchloriden, -fluoriden und -chlorfluoriden und deren Mischungen.

7. Verfahren nach Anspruch 1, bei dem der Katalysator Antimonpentachlorid ist.

8. Verfahren nach einem der Ansprüche 1 bis 7, bei dem man 0,005 bis 0,5 Mol des Katalysators pro Mol an 1,1,1,3,3-Pentachlorpropan, vorzugsweise 0,02 bis 0,25 Mol des Katalysators pro Mol an 1,1,1,3,3-Pentachlorpropan, verwendet.

9. Verfahren nach einem der Ansprüche 1 bis 8, bei dem die Reaktion bei einer Temperatur von 50 °C bis 150 °C, vorzugsweise von 80 °C bis 120 °C, durchgeführt wird.

10. Verfahren nach einem der Ansprüche 1 bis 9, bei dem man diskontinuierlich unter autogenem Druck der Reaktionsmischung verfährt.

11. Verfahren nach Anspruch 10, bei dem das Molverhältnis zwischen wasserfreier Flußsäure und 1,1,1,3,3-Pentachlorpropan zwischen 5 und 100, vorzugsweise zwischen 10 und 50, liegt.

12. Verfahren nach einem der Ansprüche 1 bis 9, bei dem man kontinuierlich unter einem Druck zwischen 5 und 60 bar, vorzugsweise zwischen 10 und 40 bar, verfährt.
